# EUROPÄISCHE PATENTSCHRIFT

(11) **EP 0 915 815 B1**
(45) Veröffentlichungstag und Bekanntmachung des Hinweises auf die Patenterteilung: **25.10.2000**
(21) Anmeldenummer: 97932712.9
(22) Anmeldetag: 25.06.1997
(51) Int. Cl.: C07C 11/24, C07C 7/12

(54) **VERFAHREN ZUR ENTFERNUNG VON SCHWEFELWASSERSTOFF, AMMONIAK, PHOSPHOR- UND ARSENWASSERSTOFF AUS ACETYLEN**
METHOD OF REMOVING HYDROGEN SULFIDE, AMMONIA, PHOSPHINE AND ARSINE FROM ACETYLENE
PROCEDE POUR ELIMINER L'ACIDE SULFHYDRIQUE, L'AMMONIAC, L'HYDROGENE PHOSPHORE ET ARSENIE CONTENUS DANS DE L'ACETYLENE

(30) Priorität: 02.07.1996 DE 19626484
(43) Veröffentlichungstag der Anmeldung: 19.05.1999
(73) Patentinhaber: Linde Technische Gase GmbH, 82049 Höllriegelskreuth (DE)
(72) Erfinder: KUNZE, Peter, D-06688 Wengelsdorf (DE); REINHARDT, Hans-Jürgen, D-06231 Bad Dürrenberg (DE); SPINDLER, Heinz-Dieter, D-06217 Merseburg (DE); WITTKE, Harald, D-06108 Halle (DE)
(74) Vertreter: Schinke, Herbert, Dr. Dr.,
(86) Internationale Anmeldenummer: DE9701334
(87) Internationale Veröffentlichungsnummer: WO9800377

(56) Entgegenhaltungen:
- PATENT ABSTRACTS OF JAPAN vol. 014, no. 208 (C-0714), 27.April 1990 & JP 02 045428 A (TOHO ASECHIREN KK), 15.Februar 1990,

## Beschreibung

Die Erfindung betrifft ein Verfahren zur Entfernung von Schwefelwasserstoff, Ammoniak, Phosphor- und Arsenwasserstoff aus Acetylen im Festbettverfahren.

Das bei der technischen Acetylenproduktion auf Basis von Calciumcarbid erzeugte Rohgas enthält insgesamt 0,05 bis 0,15 Vol.-% Schwefelwasserstoff, Ammoniak, Phosphor- und Arsenwasserstoff. Diese Verunreinigungen stören den weiteren Verarbeitungsprozeß des Acetylens und müssen daher aus dem Gas entfernt werden.

Im allgemeinen ist es ausreichend, das Acetylen bis auf einen Restgehalt an Schwefelwasserstoff, Ammoniak, Phosphor- und Arsenwasserstoff von insgesamt 0,01 Vol.-% zu reinigen. Bei bestimmten Anwendungsfällen darf der Gehalt dieser Verunreinigungen jedoch nicht über 1 Vol.-ppm liegen.

Die bekannten Reinigungsverfahren sind im allgemeinen dreistufige Prozesse, wobei meistens zunächst das Ammoniak durch Absorption in wäßrigen sauren Lösungen absorbiert und danach die in wäßrigen Lösungen nur in geringem Maße löslichen Verunreinigungen Schwefelwasserstoff, Phosphorwasserstoff und Arsenwasserstoff durch geeignete Oxidationsmittel in gut lösliche chemische Verbindungen überführt und anschließend aus dem Gasstrom entfernt werden. In der letzten Stufe erfolgt dann die Abtrennung der in der Oxidationsstufe gebildeten sauren Gase aus dem Gasstrom mittels Kalk, alkalischer wäßriger Lösungen bzw. Aktivkohle.

Die Reinigung des Rohacetylens kann auf nassem bzw. trockenem Wege erfolgen [Miller, S.A. "Acetylen" Vol. 1, Ernest Benn Limited, London, 1965 sowie Ullmanns Enzyklopädie der technischen Chemie, Vol. A1, Verlag Chemie Weinheim-New York, 1985].

Die Naßverfahren verwenden mit Füllkörpern gefüllte Waschtürme und dienen vorrangig zur Reinigung größerer Rohacetylenmengen. Zur Oxidation der Wasserstoffverbindungen des Schwefels, Phosphors und Arsens werden konzentrierte Schwefelsäure, verdünnte Salpetersäure, Natriumhypochlorid-Lösung mit einem pH-Wert, der möglichst nahe am Neutralpunkt liegt oder Chlorwasser mit einem Gehalt an freiem Chlor von 1-2 g/l verwendet.

Die Naßverfahren sind mit einer Reihe von Problemen verbunden. Bei sämtlichen Verfahren werden korrosive und zum Teil toxische Stoffe verwendet, die einen erhöhten technischen Aufwand für ein sicheres Betreiben der Reinigungsanlagen und kostenaufwendige Maßnahmen zur Entsorgung der verbrauchten Reinigungsmittel erfordern. Hinzu kommt, daß die zu entsorgenden Reinigungsmittel freie Säuren, umweltbelastende und teilweise toxische Verbindungen und bei Verwendung von Kupfer-, Silber- oder Quecksilberverbindungen hoch explosive Acetylide enthalten.

Die mit Schwefelsäure arbeitenden Naßverfahren stellen hohe Anforderungen an die Reinheit der verwendeten Schwefelsäure und sind weiterhin mit einem erhöhten technischen Aufwand zur Einhaltung der vorgegebenen Prozeßparameter verbunden. Relativ geringe Abweichungen vom geforderten Betriebsregime haben die Bildung unerwünschter Polymerisationsprodukte zur Folge bzw. verändern die vorgegebenen Reaktionszonen in der Kolonne, so daß unter bestimmten Bedingungen die austretende Säure gelöstes, nicht zu Phosphat oxidiertes Phosphin enthält, das sich beim Entspannen in die umgebende Atmosphäre entzünden kann und somit ein Sicherheitsrisiko darstellt.

Bei der Gasreinigung mit Chlorwasser oder Hypochlorit muß durch geeignete technische Maßnahmen verhindert werden, daß das mit Acetylen gesättigte und stets geringe Mengen an selbstentzündlichem Chloracetylen enthaltende Abwasser eine Explosionsgefahr für die Abwasserkanäle darstellt.

Bei geringen Mengen an zu reinigendem Rohacetylen und relativ geringen Ansprüchen an den Reinheitsgrad des gereinigten Gases arbeitet man im Festbett. Man verwendet als oxidierende Reinigungsmassen Chlorkalk, Chromsäure, Braunstein sowie Eisen(III)-chlorid und zum Ausfällen vorzugsweise Eisen-, Kupfer-, Quecksilber- und Kobaltsalze. Als Trägermaterial werden Pasten, Asche, Kieselgur, Perlitsand oder keramische Materialien verwendet [SU 1467080].

Mit den bekannten Trocken- bzw. Festbettverfahren können die Nachteile der Naßverfahren, insbesondere in bezug auf die Korrosions- und Explosionsgefahr sowie auf die Entsorgung umweltbelastender und teilweise toxischer Stoffe, nicht beseitigt werden. Hinzu kommt, daß die im Festbett arbeitenden Verfahren mit einem hohen spezifischen Verbrauch an Reinigungsmitteln verbunden sind.

Je nach zu entfernender Menge an Verunreinigungen und Reinigungstiefe sind bis zu 50 kg Reinigungsmittel/100 m³ Rohacetylen erforderlich. Weiterhin ist von Nachteil, daß auf Grund der Feuchtigkeitszunahme im Festbett im Laufe des Betriebes der Druckverlust ansteigt, was zu einer weiteren Einschränkung der Betriebsdauer des Reinigungsmittels führt.

Zur Reduzierung des spezifischen Verbrauchs an Reinigungsmitteln wurde vorgeschlagen, als Tägersubstanz Zeolithe bzw. Aktivkohlen einzusetzen, die durch mineralische Säuren [SU1181692], Aluminiumchlorid [SU 1142143A] bzw. Kupfer(II)-, Kaliumtetrachlorpalladium(II)- und Eisen(III)-chlorid [SU 1028350] modifiziert wurden.

Diese Festbettverfahren haben den Nachteil, daß mit der erreichbaren Verringerung des spezifischen Verbrauchs an Reinigungsmasse die Mehrkosten für die Trägersubstanz nicht gedeckt werden können. Hinzu kommt, daß das Verfahren gemäß SU 1181692 zusätzliche Aufwendungen für die Einhaltung eines bestimmten Feuchtegrades der Aktivkohle während des Reinigungsprozesses erfordert. Beim Verfahren gemäß SU 1028350 besteht durch die Verwendung von Kupfer(II)-chlorid die Gefahr der Bildung von hochexplosiblem Kupferacetylid.

In JP-A-2 045 428 ist ein Verfahren beschrieben zur Entfernung von Phosphorwasserstoff aus Acetylen in dem mit Sauerstoff aktivierter Kohlenstoff als Adsorptionsmittel verwendet wird.

Das Ziel der Erfindung ist ein wirtschaftliches Verfahren zur Entfernung von Schwefelwasserstoff, Ammoniak, Phosphor- und Arsenwasserstoff aus Acetylen, das die Nachteile bekannter Verfahren ganz oder teilweise beseitigt.

Es bestand die Aufgabe, die im Rohacetylen enthaltenen Verunreinigungen Schwefelwassserstoff, Ammoniak, Phosphor- und Arsenwasserstoff in einem Festbettverfahren bis auf Spuren aus dem Rohgas zu entfernen.

Diese Aufgabe wird erfindungsgemäß durch ein Verfahren zur Entfernung von Schwefelwasserstoff, Ammoniak, Phosphor- und Arsenwasserstoff aus Acetylen im Festbettverfahren gelöst, bei dem das zu reinigende Acetylen in einem ein- oder mehrstufigen Prozeß durch aus mehreren Schichten bestehende Aktivkohleschüttungen geleitet wird, wobei die Aktivkohle einmal mit Kaliumjodid modifiziert, einmal mit Zinksulfat modifiziert und einmal mit Ammoniak aktiviert wurde.

Die Anlage sollte vorzugsweise so aufgebaut sein, daß das zu reinigende Acetylen zunächst die mit Kaliumjodid, danach die mit Zinksulfat modifizierte und anschliessend die unter Ammoniakatmosphäre aktivierte Aktivkohle durchströmt.

Die einzusetzenden Volumina der Aktivkohleschüttungen hängen vom Verhältnis der Verunreinigungen ab. Die mit Kaliumjodid und Zinksulfat modifizierten Aktivkohlen sollten zweckmäßig im Volumenverhältnis von 2:1 bis 1:2 (vorzugsweise 1:1) eingesetzt werden und die unter Ammoniakatmosphäre aktivierte Aktivkohle zweckmäßig im Volumenverhältnis von 0,4:1 bis 1,5:1 (vorzugsweise 0,8:1) zur Summe der mit Kaliumjodid und Zink-sulfat modifizierten Aktivkohlen.

Die mit Kaliumjodid modifizierte Aktivkohle enthält zweckmäßig einen Kaliumjodidgehalt von 2 bis 10 Ma-%, vorzugsweise etwa 5 Ma-%.

Vorteilhafterweise sollte die mit Zinksulfat modifizierte Aktivkohle einen Zinksulfatgehalt von 10 bis 30 Ma-%, vorzugsweise etwa 20 Ma-%, aufweisen. Die mit Ammoniak aktivierte Aktivkohle sollte zweckmäßig einen Stickstoffgehalt von 0,5 bis 1,5 Ma-%, vorzugsweise etwa 1,5 Ma-%, aufweisen.

Das erfindungsgemäße Verfahren weist gegenüber den bekannten Verfahren wesentliche Vorteile auf. Es ist vergleichsweise einfach aufgebaut und stellt keine erhöhten Anforderungen an die Apparate sowie an die Meß- und Regeleinrichtungen. Die einzusetzenden Werkstoffe entsprechen dem für Adsorptionsprozesse üblichen Standard. Das Verfahren arbeitet bei Normaldruck und Umgebungstemperatur und ist durch einen geringen Druckverlust gekennzeichnet, so daß keine zusätzlichen Aufwendungen zur Förderung des Gases erforderlich sind.

Beim erfindungsgemäßen Verfahren treten keine unerwünschten Nebenreaktionen auf, die die Acetylenausbeute verringern bzw. die Leistungsfähigkeit der Aktivkohle vermindern. Die durch den Reinigungsprozeß verbrauchten Aktivkohlen können problemlos entsorgt werden.

Von besonderem Vorteil ist der geringe spezifische Verbrauch an Reinigungsmasse. Mit 1 kg Reinigungsmasse können bis zu 105 m³ Rohacetylen bis auf einen Restgehalt an Schwefelwasserstoff, Ammoniak, Phosphor- und Arsenwasserstoff von insgesamt 10 Vol.-ppm gereinigt werden.

Die Erfindung wird an folgendem Ausführungsbeispiel näher erläutert:

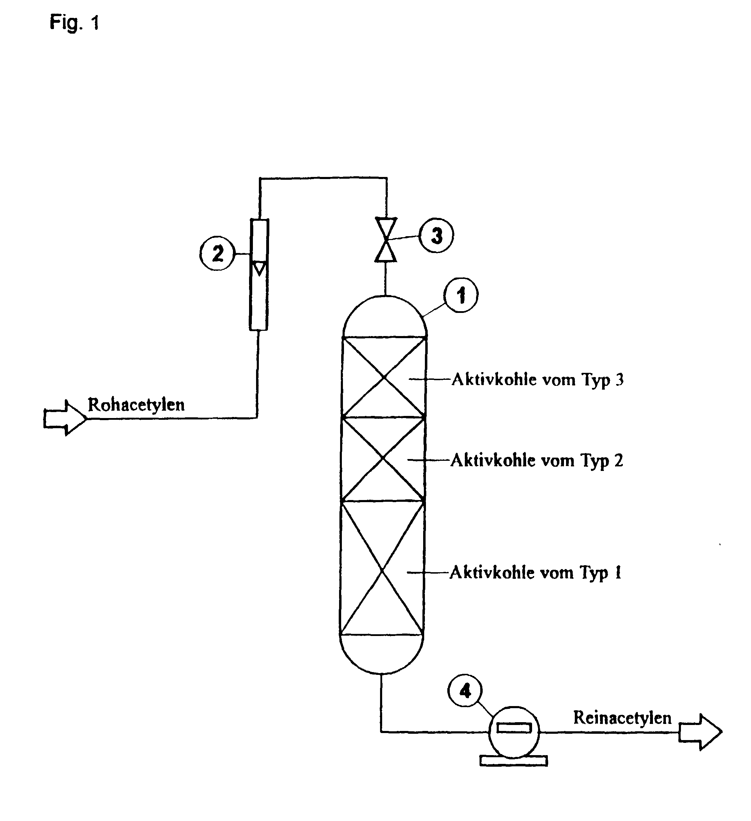

Die Untersuchungen erfolgten in der in Fig. 1 schematisch dargestellten Laborapparatur; darin bedeuten
1 Glassäule
2 Schwebekörpermesser
3 Ventil
4 Gasuhr

In der Glassäule 1 mit einem Innendurchmesser von 27 mm und einer Gesamtlänge von 1000 mm wurden nacheinander
- 200 ml Aktivkohle vom Typ 1 - mit Ammoniak aktiviert -
- 125 ml Aktivkohle vom Typ 2 - mit Zinksulfat modifiziert - und
- 125 ml Aktivkohle vom Typ 3 - mit Kaliumjodid modifiziert -
eingefüllt. Die verwendeten Aktivkohlen sind in Tabelle 1 näher charakterisiert.

Anschließend wurden durch diese Aktivkohleschüttung säulenabwärts ca. 0,1 m³i.N./h Rohacetylen mit den in Tabelle 2 angegebenen durchschnittlichen Parametern geleitet. Die Einstellung der Gasmenge erfolgte mit dem Ventil 3 anhand des mit dem Schwebekörpermesser 3 angezeigten Volumenstromes.

Das der Säule entströmende Reinacetylen wurde über die Gasuhr 4 zur Atmosphäre abgeleitet und in bestimmten Zeitabständen dessen Gehalt an Schwefelwasserstoff, Ammoniak, Phosphor- und Arsenwasserstoff bestimmt. Die erhaltenen Ergebnisse sind in Tabelle 3 zusammengestellt.

**Tabelle 1:**

| Charakteristik der verwendeten Aktivkohletypen | | | | |
|---|---|---|---|---|
| Benennung | Dimension | Zahlenwert | | |
| | | Typ 1 | Typ 2 | Typ 3 |
| Strangdurchmesser | mm | - | ca. 1 | ca. 2,9 |
| Stranglänge | mm | - | 2-4 | 4-9 |
| Komdurchmesser | mm | 1-3 | - | - |
| Schüttgewicht | kg/m³ | 581 | 512 | 463 |
| Gehalt an: | | | | |
| Kohlenstoff | Ma-% | 87,5 | 64,6 | 85,8 |
| Sauerstoff | Ma-% | 4,1 | 19,5 | 4,0 |
| Schwefel | Ma-% | - | 4,0 | - |
| Stickstoff | Ma-% | 1,1 | 0,3 | 0,4 |
| Jod | Ma-% | - | - | 4,2 |
| Kalium | Ma-% | - | 0,2 | 1,2 |
| Zink | Ma-% | - | 8,2 | - |

**Tabelle 2:**

| Durchschnittliche Parameter des Rohacetylens | | |
|---|---|---|
| Benennung | Dimension | Zahlenwert |
| Druck | kPa, abs. | 101 |
| Temperatur | °C | 24 |
| Gehalt an | | |
| Wasserdampf | Vol.-% | 2,4 |
| Sauerstoff | Vol.-ppm | 1200 |
| Schwefelwasserstoff | Vol.-ppm | 80 |
| Ammoniak | Vol.-ppm | 400 |
| Phosphorwasserstoff | Vol.-ppm | 300 |
| Arsenwasserstoff | Vol.-ppm | 3 |

**Tabelle 3:**

| Durchschnittlicher Gehalt an Verunreinigungen im gereinigten Acetylen in Abhängigkeit von der durchgesetzten Gasmenge | | | | |
|---|---|---|---|---|
| Gasdurchsatz in m³i.N. | Gehalt an Verunreinigungen in Vol.-ppm | | | |
| | H₂S | NH₃ | PH₃ | AsH₃ |
| 10,0 | <1 | <1 | <1 | <1 |
| 15,0 | <1 | <1 | <1 | <1 |
| 20,0 | <1 | <1 | <1 | <1 |
| 22,0 | <1 | 1 | 1 | <1 |
| 24,0 | 1 | 2 | 3 | <1 |
| 25,3 | 1 | 4 | 4 | <1 |

## Patentansprüche

1. Verfahren zur Entfernung von Schwefelwasserstoff, Ammoniak, Phosphor- und Arsenwasserstoff aus Acetylen im Festbettverfahren, dadurch gekennzeichnet, daß das zu reinigende Acetylen in einem ein- oder mehrstufigen Prozeß durch aus mehreren Schichten bestehende Aktivkohleschüttungen geleitet wird, wobei die Aktivkohle einmal mit Kaliumjodid modifiziert, einmal mit Zinksulfat modifiziert und einmal mit Ammoniak aktiviert wurde.

2. Verfahren nach Anspruch 1, dadurch gekennzeichnet, daß das zu reinigende Acetylen zunächst die mit Kaliumjodid, danach die mit Zinksulfat modifizierte und anschließend die unter Ammoniakatmosphäre aktivierte Aktivkohle durchströmt.

3. Verfahren nach Anspruch 1 und 2, dadurch gekennzeichnet, daß die mit Kaliumjodid und Zinksulfat modifizierten Aktivkohlen im Volumenverhältnis von 2:1 bis 1:2, vorzugsweise 1:1, eingesetzt werden und die unter Ammoniakatmosphäre aktivierte Aktivkohle im Volumenverhältnis von 0,4:1 bis 1,5:1, vorzugsweise 0,8:1, zur Summe der mit Kaliumjodid und Zinksulfat modifizierten Aktivkohlen verwendet wird.

4. Verfahren nach Anspruch 1 bis 3, dadurch gekennzeichnet, daß die mit Kaliumjodid modifizierte Aktivkohle einen Kaliumjodidgehalt von 2 bis 10 Ma-%, vorzugsweise etwa 5 Ma-%, aufweist.

5. Verfahren nach Anspruch 1 bis 3, dadurch gekennzeichnet, daß die mit Zinksulfat modifizierte Aktivkohle einen Zinksulfatgehalt von 10 bis 30 Ma-%, vorzugsweise etwa 20 Ma-%, aufweist.

6. Verfahren nach Anspruch 1 bis 3, dadurch gekennzeichnet, daß die mit Ammoniak aktivierte Aktivkohle einen Stickstoffgehalt von 0,5 bis 1,5 Ma-%, vorzugsweise etwa 1,5 Ma-%, aufweist.

## Claims

1. Method of removing hydrogen sulfide, ammonia, phosphine and arsine from acetylene in a fixed-bed process, characterized in that the acetylene to be purified is guided through packed beds comprising several layers of activated carbon, with the different layers being modified by potassium iodide, zinc sulfate, and ammonia, respectively.

2. Method according to claim no. 1, characterized in that the acetylene to be purified flows first through the potassium iodide modified activated carbon, then through the zinc sulfate modified activated carbon, and finally through the charcoal layer activated under an ammonia atmosphere.

3. Method according to claims nos. 1 and 2, characterized in that the potassium iodide and zinc sulfate modified activated carbon is employed in volumetric ratios ranging from 2 : 1 to 1 : 2, preferably 1 : 1, and that the charcoal activated under an ammonia atmosphere and the total volume of potassium iodide and zinc sulfate modified activated carbon is in volumetric ratios ranging from 0.4 : 1 to 1.5 : 1, preferably 0.8 : 1.

4. Method according to claims nos. 1 through to and inclusive 3, character' ized in that the potassium iodide modified activated carbon has a potassium iodide content of 2 per cent to 10 per cent in weight, preferably approx. 5 per cent in weight.

5. Method according to claims nos. 1 through to and inclusive 3, characterized in that the zinc sulfate modified activated carbon has a zinc sulfate content of 10 per cent to 30 per cent in weight, preferably approx. 20 per cent in weight.

6. Method according to claims nos. 1 through to and inclusive 3, characterized in that the ammonia activated charcoal has a nitrogen content of 0.5 per cent to 1.5 per cent in weight, preferably approx. 1.5 per cent in weight.

## Revendications

1. Procédé d'élimination de l'acide sulfhydrique, de l'ammoniac, de l'hydrogène phosphoré et de l'hydrogène arsénié de l'acétylène dans le procédé de lit fixe, **caractérisé par le fait que** l'acétylène devant être épuré est guidé au cours d'un processus en une ou plusieurs étapes par des charges de charbon actif se composant de plusieurs couches, le charbon actif étant modifié une fois à l'aide de iodure de potassium, une fois à l'aide de sulfate de zinc et étant activé une fois avec de l'ammoniac.

2. Procédé selon la revendication 1, **caractérisé par le fait que** l'acétylène devant être épuré parcourt en premier lieu le charbon actif modifié à l'aide de iodure de potassium, ensuite le charbon actif modifié à l'aide de sulfate de zinc, et pour finir, le charbon actif activé sous atmosphère ammoniaquée.

3. Procédé selon la revendication 1 ou 2, **caractérisé par le fait que** les charbons actifs modifiés à l'aide de iodure de potassium et de sulfate de zinc sont mis en application dans un rapport volumique allant de 2:1 à 1:2, de préférence 1:1 et le charbon actif activé sous atmosphère ammoniaquée est utilisé dans un rapport volumique allant de 0,4:1 à 1,5:1, de préférence 0,8:1, calculé par rapport à la somme des charbons actifs modifiés à l'aide de iodure de potassium et de sulfate de zinc.

4. Procédé selon la revendication 1 à 3, **caractérisé par le fait que** le charbon actif modifié à l'aide de iodure de potassium présente une teneur en iodure de potassium allant de 2 à 10 % de la masse, de préférence environ 5 % de la masse.

5. Procédé selon la revendication 1 à 3, **caractérisé par le fait que** le charbon actif modifié à l'aide de sulfate de zinc présente une teneur en sulfate de zinc allant de 10 à 30 % de la masse, de préférence environ 20 % de la masse.

6. Procédé selon la revendication 1 à 3, **caractérisé par le fait que** le charbon actif activé à l'aide d'ammoniac présente une teneur en azote allant de 0,5 à 1,5 % de la masse, de préférence environ 1,5 % de la masse.
